# EUROPEAN PATENT APPLICATION

(11) **EP 4 368 698 A1**
(43) Date of publication of application: **15.05.2024**
(21) Application number: 22211936.4
(22) Date of filing: 07.12.2022
(51) Int. Cl.: C12N 5/00, C12N 5/078, C12N 5/0789

(54) **OPTIMIZED CELL CULTURE MEDIUM UTILIZING IRON (III) CITRATE AS AN IRON DELIVERY METHOD FOR THE IN VITRO, BIOREACTOR-CENTRIC PRODUCTION OF MANUFACTURED BLOOD**

(30) Priority: 09.11.2022 US 202263382998 P
(71) Applicant: Sciperio, Inc., Orlando, Florida 32826 (US)
(72) Inventor: Sasserath, Trevor, Orlando (US); Moser, Janice M., Oviedo (US); Haupfear, Kelly, Titusville (US); Perkowski, Casey W., Winter Park (US); McDowell, Evan, Orlando (US); Church, Kenneth H., Orlando (US)
(74) Representative: Boult Wade Tennant LLP

(57) **Abstract**

A formulation for a culture medium that is specifically designed to supplement a culture of differentiating hematopoietic stem cells (HSCs) with the factors necessary for high density manufacture of red blood cells (RBCs) while only requiring partial medium exchanges on a periodic basis. Further, the present disclosure identifies a factor, iron (III) citrate, also referred to as ferric citrate, that can supplant more commonly utilized sources of biological iron in culture medium for a more stable, more cost-effective medium formulation for superior vertical scalability.

## Description

### GRANT REFERENCE

This invention was made with government support under Contract No. HU0001-20-2-0011 awarded by the Department of Defense, an agency of the U.S. Government. The government has certain rights in the invention.

### FIELD OF THE INVENTION

The present invention relates to biological manufacturing and in vitro cell culture. More particularly, but not exclusively, the present invention relates to an optimized cell culture medium for use in in vitro, bioreactor-centric production of manufactured blood.

### BACKGROUND

Advancements to in vitro culture methodologies have paved the way for the implementation of Good Manufacturing Practices (GMP) culture methods for the bulk manufacture of biological therapeutics, such as manufactured blood products, including red blood cells. In order to generate red blood cells efficiently in an in vitro setting, a defined, optimized culture medium that supplies the factors necessary to foster cell growth and proliferation while allowing for the maintenance of a homeostatic culture environment is required.

### SUMMARY

Therefore, it is a primary object, feature, or advantage of the present invention to improve over the state of the art.

Another object, feature, or advantage is to provide a culture medium suitable for use in the bulk manufacture of biological therapeutics.

It is a further object, feature, or advantage to provide a defined, optimized culture medium that supplies the factors necessary to foster cell growth and proliferation while allowing for the maintenance of a homeostatic culture environment.

It is a still further object, feature, or advantage to provide a medium that is beneficial in increasing the proliferative capacity of differentiating hematopoietic stem cells.

Another object, feature, or advantage is to provide a medium with a non-proteinaceous iron source which is thermally stable.

Yet another object, feature, or advantage is to provide a medium with a non-proteinaceous iron source which is cost effective.

It is a still further object, feature, or advantage of the present invention to provide medium for a bioreactor configured to provide periodic partial medium exchanges.

Another object, feature, or advantage is to provide a medium optimized for red blood cell (RBC) differentiation.

Yet another object, feature, or advantage is to provide a medium which avoids the labor-intensive process of holo-transferrin synthesis and characterization, as well as its thermally labile nature which leads to higher material costs.

One or more of these and/or other objects, features, or advantages of the present invention will become apparent from the specification and claims that follow.

No single embodiment need provide each and every object, feature, or advantage. Different embodiments may have different objects, features, or advantages. Therefore, the present invention is not to be limited to or by any objects, features, or advantages stated herein.

According to one aspect a medium for culturing cells optimized for use in a bioreactor with periodic partial medium exchanges is provided. The medium includes iron (III) citrate at a concentration of between 1 µg/mL and 500 µg/mL. The medium need not contain holo-transferrin and may include a plurality of components including amino acids and vitamins having concentrations optimized for in vitro bioreactor-based manufacture of therapeutics which may be cellular in nature such as red blood cells (RBCs) or biologics. A system may include the bioreactor with a recirculating loop and the medium.

According to another aspects, a medium for culturing cells optimized for use in a bioreactor with periodic partial medium exchanges or additions is provided, the medium including a plurality of medium components optimized for in vitro bioreactor-based manufacture of red blood cells (RBCs). The medium may include holo-transferrin at a concentration between about 0.68 mg/mL and 0.95 mg/mL or may include iron (III) citrate at a concentration of between 1 µg/mL and 500 µg/mL. A system may include a bioreactor with the medium and the bioreactor may have a recirculating loop and/or may be an expandable bioreactor.

According to another aspect, a method of formulating a medium for use in a bioreactor with periodic partial medium exchanges or additions. The method may include determining average depletion rates for a plurality of factors in the medium when used in the bioreactor with partial medium exchanges and formulating the medium based on the average depletion rates to supply the plurality of factors to allow for cell growth and proliferation while maintaining a homeostatic culture environment. The medium may include iron (III) citrate at a concentration of between 1 µg/mL and 500 µg/mL.

### BRIEF DESCRIPTION OF THE DRAWINGS

Illustrated embodiments of the disclosure are described in detail below with reference to the attached drawing figures, which are incorporated by reference herein.

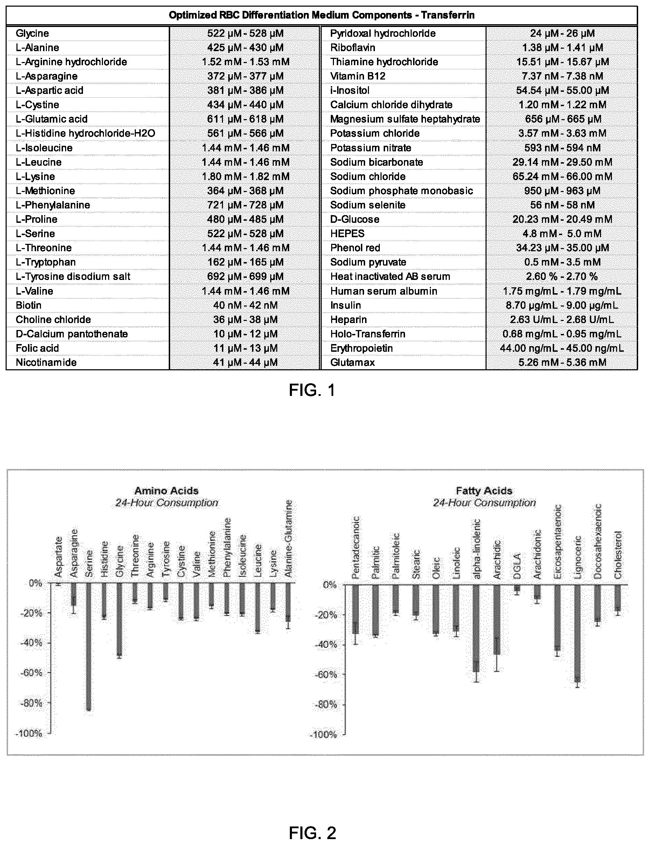

FIG. 1 is a table showing a concentration range for each factor in an example of an optimized RBC differentiation medium: 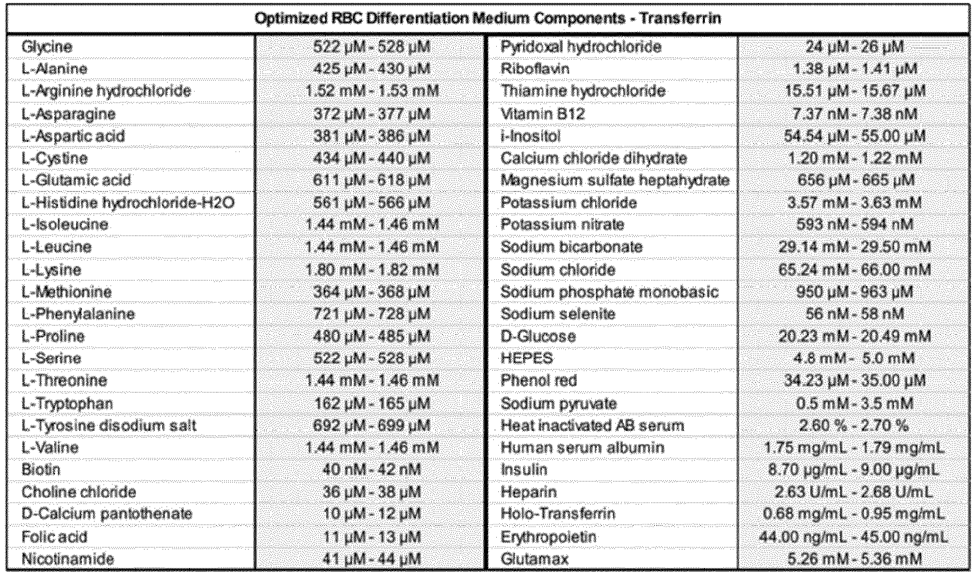
FIG. 2 is an average 24-hour depletion of amino acids and fatty acids from culture medium by differentiating HSCs.

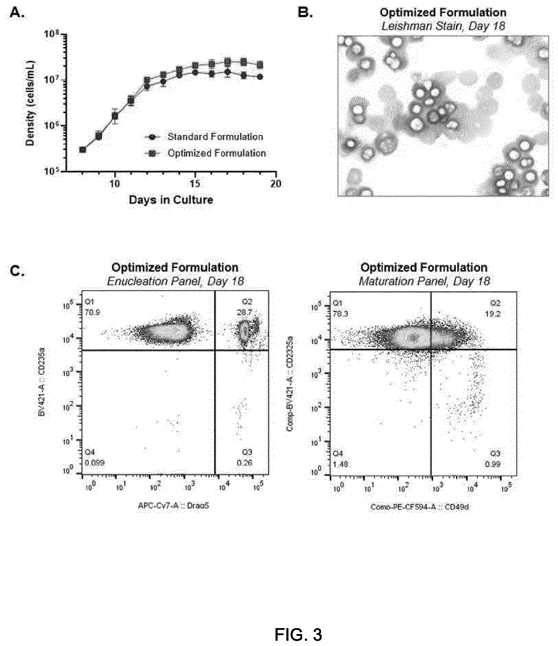

FIG. 3 illustrates optimized RBC differentiation medium provides a proliferative advantage to cultures of HSCs, as is demonstrated by (A) relative increases in culture density over time. (B) RBCs manufactured using optimized formulation maintain a normal morphology, (C) exhibit sufficient enucleation (DRAQ5-, CD235a+), and (D) express markers characteristic of mature RBCs (CD235a+, CD49d-).

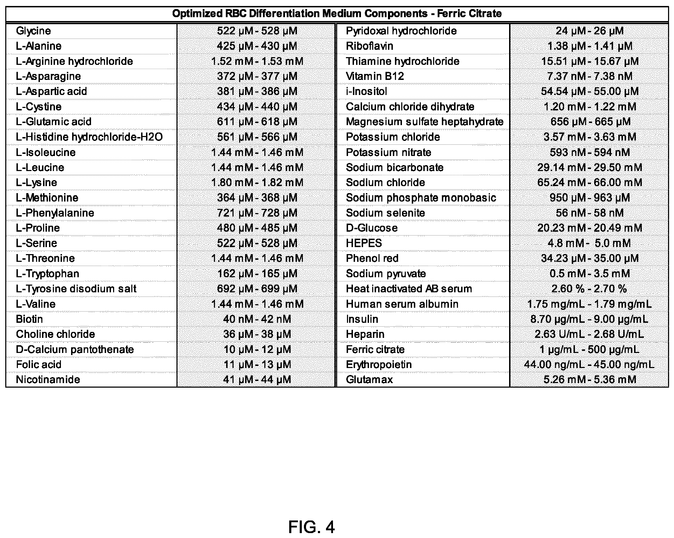

FIG. 4 is a table showing a concentration range for each factor in optimized RBC differentiation medium, with ferric citrate serving as the primary source of iron: 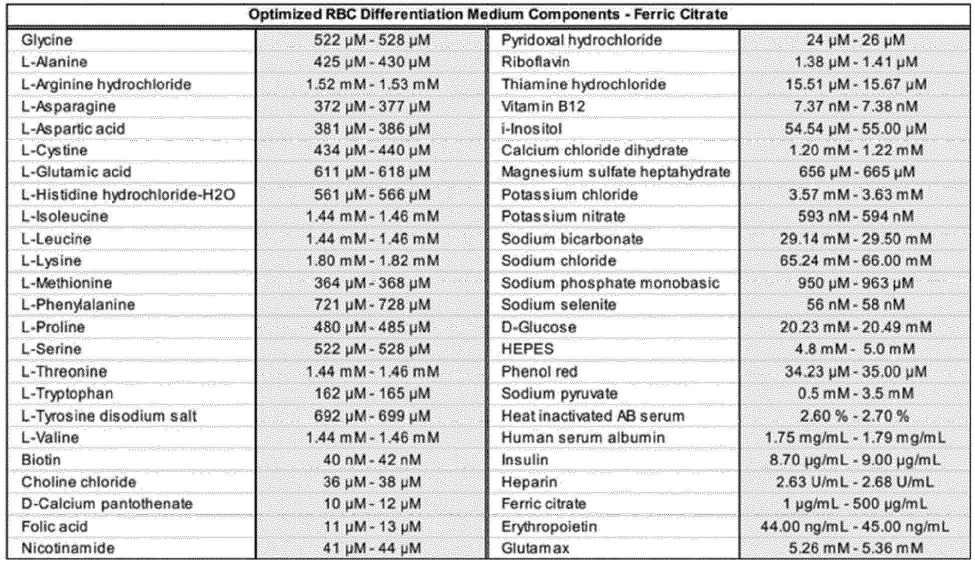

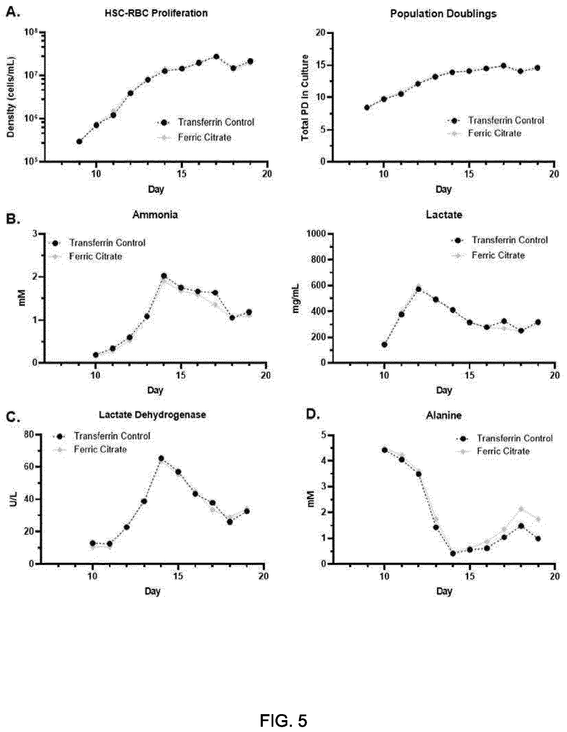

FIG. 5 illustrates that ferric citrate can be substituted for holo-transferrin in high density differentiation of HSCs into RBCs. (A) Ferric citrate has a comparable effect on HSC density and cumulative population doubling to transferrin. (B) Secretion of cellular waste compounds ammonia and lactate is equivalent between ferric citrate and transferrin, indicating similar metabolic rates. (C) Rates of lytic cell death are similar between cultures treated with ferric citrate and transferrin, as is indicated by the release of lactate dehydrogenase (LDH). (D) Amino acid consumption is similar between the two conditions, as is represented by the consumption of alanine and other amino acids.

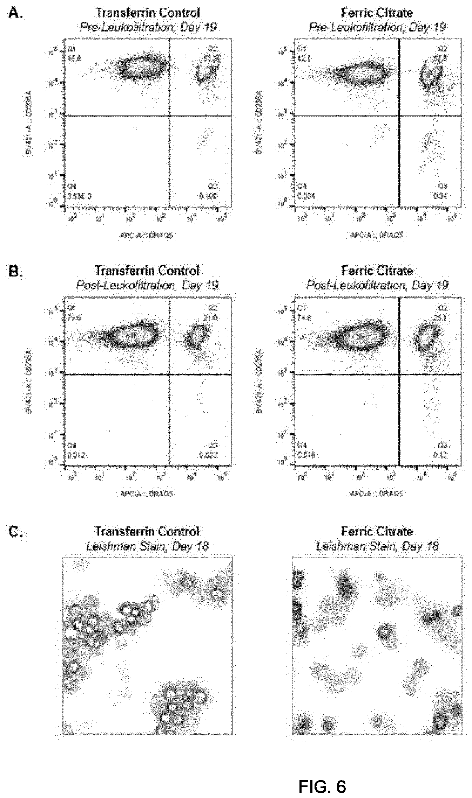

FIG. 6 illustrates ferric citrate-treated RBCs enucleate at rates similar to transferrin-treated RBCs. (A) Following differentiation in optimized medium with either transferrin or ferric citrate, there is only a ~4% difference in the overall enucleation by these RBCs. (B) Concentration of enucleated cells by leukofiltration increases the concentration of transferrin and ferric citrate treated cells to 79% and 74.8%, respectively. (C) Cells differentiated with transferrin and ferric citrate are morphologically similar, as is observable by Leishman stain.

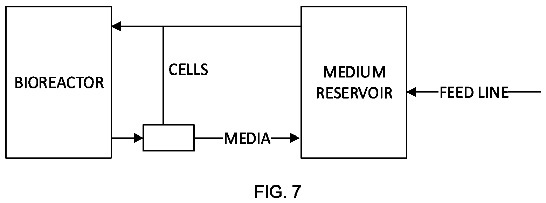

FIG.7 illustrates one example of a bioreactor with a recirculating loop.

### DETAILED DESCRIPTION

### Overview

A novel culture medium is provided which is specifically designed to supplement a high-density culture of differentiating hematopoietic stem cells in a bioreactor with the factors necessary to manufacture of red blood cells with periodic partial medium exchanges or additions. Further, a factor, iron (III) citrate, also referred to as ferric citrate, is identified that can supplant more commonly utilized sources of biological iron in culture medium for a more stable, more cost-effective medium formulation for superior vertical scalability in a bioreactor.

As such, according to one embodiment an optimized cell culture medium is provided that contains elevated amino acid/vitamin concentrations specific to the optimized in vitro bioreactor-based manufacture of RBCs. According to another embodiment iron (III) citrate is identified as a replacement for proteinaceous sources of biological iron delivery.

In vitro human cell culture has become the most desirable mode of biological research in the 21st century, due to relative ease of experimentation, ethical concerns surrounding experimentation on laboratory animals, and improved translation of findings from the bench towards clinical applications. For these reasons and others, developments in in vitro technologies have shifted the global paradigm of biological research away from animal studies and towards development of in vitro culture methodologies for pharmaceutical development and mechanistic studies. Such advancements to in vitro culture methodologies have paved the way for the implementation of GMP culture methods for the bulk manufacture of biological therapeutics. These therapeutics can be cellular in nature, as in the case of manufactured red blood cells, or can be biologics derived from living cells, such as erythropoietin or other recombinant proteins. In either case, a defined, optimized culture medium that supplies the factors necessary to foster cell growth and proliferation while allowing for the maintenance of a homeostatic culture environment is required. Following optimization, utilization of this culture medium should allow the culture to maximally produce the desired product.

Growth factors, including various essential and nonessential amino acids, vitamins, fatty acids, metals, and proteins, are consumed by cells during the normal metabolic processes that proceed during their life cycles. The consumption profiles for each factor vary considerably between cell lineages, maturation timelines, and density of culture. As such, existing medium formulations must be adapted to meet the cells' metabolic needs as defined by the rates at which they consume each factor. Additionally, the method used to cultivate these cells can affect the rate of factor consumption. Bioreactors in particular are designed to maintain stable homeostatic conditions in which cells can proliferate maximally and efficiently, increasing total metabolic rate of the system and increasing demand for certain amino acids, vitamins, and fatty acids, among other factors. Due to the high culture volume native to most bioreactors, and subsequently the high volume of medium required to culture cells in them, an optimized, high-nutrient feed becomes necessary to reduce overall medium usage and keep operational costs in a reasonable range. To this end, the present disclosure provides a formulation for a novel culture medium that is specifically designed to supplement a culture of differentiating hematopoietic stem cells (HSCs) with the factors necessary for high density manufacture of red blood cells (RBCs) while only requiring partial medium exchanges on a periodic basis. Further, the present disclosure identifies a factor, iron (III) citrate, also referred to as ferric citrate, that can supplant more commonly utilized sources of biological iron in culture medium for a more stable, more cost-effective medium formulation for superior vertical scalability. As such, an optimized cell culture medium is provided.

According to one aspect, elevated amino acid/vitamin concentrations specific to the optimized in vitro manufacture of RBCs are provided.

According to another aspect, a medium is provided which includes Iron (III) citrate as a replacement for proteinaceous sources of biological iron delivery.

### Component Description

### Elevated amino acid/vitamin concentrations specific to the optimized in vitro bioreactor-based manufacture of RBCs.

The medium formulation has specifically been designed for the efficient, high density, in vitro manufacture of RBCs from HSCs in bioreactors. It is formulated to optimally supply certain growth factors at elevated concentrations for rapid HSC expansion and mediated erythropoiesis using a bioreactor (Figure 1). The formulation has been determined experimentally using the average depletion rates of various factors by differentiating HSCs in high-density culture (Figure 2), and can be employed in the production of manufactured RBCs both in static culture (e.g. culture flasks) and in continually agitated suspension culture (e.g. bioreactors).

Evaluation of the efficacy of this optimized culture medium formulation demonstrates that targeted supplementation of the factors listed in Figure 1 results in a 100% increase in culture density over a prolonged culture period while maintaining normal individual cell morphology and normal capacity for enucleation and maturation (Figure 3).

### Ferric citrate as an alternative iron source

Specifically in the case of HSC culture protocols, traditional medium formulations utilize the iron-loaded protein holo-transferrin to supply their cells with a biologically available source of iron, as it is the primary method of in vivo iron delivery to differentiating erythrocytes. This avenue of iron delivery has been proven to be effective in small-scale culture on multiple occasions, but suffers from numerous drawbacks that complicate its implementation in bulk cellular manufacturing operations. Specifically, holo-transferrin possesses multiple iron-binding domains, and their chemical structure mandates that specific environmental conditions be met for each domain to effectively bind iron. These conditions are easily attainable in vivo and result in cyclical processes of transferrin depletion and replenishment that ensures efficient delivery of iron to cells in need. However, as transferrin is not easily replenished in vitro, once transferrin offloads its ferric iron payload and is ejected from the cell as apo-transferrin it effectively becomes an extracellular waste product that contributes to osmolality creep and can upset the osmotic balance of the culture medium as more holo-transferrin is added to replace it. This can interfere with the establishment of a high-density culture due to the resulting osmotic incompatibility, and a truly optimized medium should aim to mitigate such problematic factors. Additionally, the labor-intensive process of holo-transferrin synthesis and characterization, as well as its thermally labile nature, leads to higher material costs when used as a medium supplement for in vitro cell culture.

Citrates are compounds that are produced by plants and are secreted through the roots to aid in uptake up inorganic compounds, such as metals. These citrates chelate trivalent Fe3+ to aid in reabsorption and to prevent precipitation, and the resulting iron (III) citrate is transported through the xylem to the leaves where it is photoreduced to Fe2+, is decoupled from the accompanying citrate, and utilized in various biological processes. These ferric citrates can be synthesized easily in a laboratory setting and utilized in mammalian cultures to accomplish similar goals of iron delivery, to the extent that ferric citrate can be added to the culture medium and be accessed by cells with high iron demand for incorporation into normal metabolic processes. Here, ferric citrate as an adequate substitute for holo-transferrin in a cell culture medium that facilitates the differentiation of HSCs into RBCs. Such an application of ferric citrate as a transferrin replacement is nonobvious, as it is a less-efficient method of iron delivery. However, the relative reduction in efficiency is less crucial in the context of RBC culture in a modular and expandable bioreactor where vertical scalability is mediated by medium cost and stability.

The present inventors have experimentally determined that a concentration range between 1 µg/mL and 500 µg/mL is effective in driving directed HSC differentiation through the stages of erythropoiesis in a manner that is equivalent to that of holo-transferrin (Figure 4).

Analysis of the subsequent culture medium indicates that substitution of ferric citrate for transferrin results in comparable cell proliferation trends, as well as maintaining similar rates of metabolic waste production, lytic cell death, and amino acid consumption (Figure 5).

RBCs that result from ferric citrate-laden differentiation medium have been demonstrated to enucleate and mature at rates analogous to RBCs differentiated with transferrin, as well as retain the normal enucleated, biconcave morphologies characteristic of mature erythrocytes (Figure 6).

Figure 7 is a block diagram illustrating one example of a bioreactor with a re-circulating loop. In the example shown, cells may be separated from media. The media may flow to a medium reservoir. There may be a feed line present to so that additional components can be added to the medium reservoir. After cells are separated from the media, the cells may be returned to the bioreactor and medium from the medium reservoir may also be returned to the bioreactor. Thus, as shown, the bioreactor provides for periodic partial medium additions or exchanges. It is to be understood that other configurations of bioreactors may be used which otherwise provide for periodic partial medium exchanges. In another example, the bioreactor may have an expandable volume such as that shown and described in U.S. Published Patent Application No. 2022/0177822. Therefore, methods, compositions, and systems have been shown and described for use of media such as in a bioreactor, and especially a bioreactor with a re-circulating loop and/or with an expandable volume.

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

As used herein, and unless otherwise indicated, the term "about" or "approximately" means an acceptable error for a particular value as determined by one of ordinary skill in the art, which depends in part on how the value is measured or determined.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and subrange is explicitly recited. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges such as from 1-3, from 2-4, and from 3-5, etc., as well as 1, 2, 3, 4, and 5, individually. This same principle applies to ranges reciting only one numerical value as a minimum or a maximum. Furthermore, such an interpretation should apply regardless of the breadth of the range or the characteristics being described.

The abbreviation, "e.g." is derived from the Latin exempli gratia, and is used herein to indicate a non-limiting example. Thus, the abbreviation "e.g." is synonymous with the term "for example".

The invention is not to be limited to the particular embodiments described herein. In particular, the invention contemplates numerous variations in the structure of the bioreactor, the type of therapeutics such as those which are cellular in nature such as manufactured red blood cells or type of biologic derived from living cells such as erythropoietin or other recombinant proteins. The foregoing description has been presented for purposes of illustration and description. It is not intended to be an exhaustive list or limit any of the invention to the precise forms disclosed. It is contemplated that other alternatives or exemplary aspects are considered included in the invention. The description is merely examples of embodiments, processes, or methods of the invention. It is understood that any other modifications, substitutions, and/or additions can be made, which are within the intended spirit and scope of the invention.

## Claims

1. A medium for culturing cells optimized for use in a bioreactor with periodic partial medium exchanges, the medium comprising iron (III) citrate at a concentration of between 1 µg/mL and 500 µg/mL.

2. The medium of claim 1 wherein the medium does not contain holo-transferrin.

3. The medium of claim 1 or claim 2 further comprising a plurality of components including amino acids and vitamins having concentrations optimized for in vitro bioreactor-based manufacture of therapeutics.

4. The medium of claim 3 wherein the therapeutics are cellular in nature.

5. The medium of claim 4 wherein the therapeutics are red blood cells (RBCs).

6. The medium of claim 3 wherein the plurality of amino acids comprises:
glycine at a concentration of between about 522 µM and 528 µM;
L-alanine at a concentration of between about 425 µM and 430 µM;
L-arginine hydrochloride of between about 1.52 mM and 1.53 mM;
L-asparagine of between about 372 µM and 377 µM;
L-aspartic acid of between about 381 µM and 386 µM;
L-cystine of between about 434 µM and 440 µM;
L-glutamic acid of between about 611 µM and 618 µM;
L-histidine hydrochloride-H2O of between about 561 µM and 566 µM;
L-isoleucine at a concentration between about 1.44 mM and about 1.46 mM;
L-leucine at a concentration between about 1.44 mM and about 1.46 mM;
L-lysine at a concentration between about 1.80 mM and about 1.82 mM;
L-methionine at a concentration between about 364 µM and 368 µM;
L-phenylalanine at a concentration between about 721 µM and 728 µM;
L-proline at a concentration between about 480 µM and 485 µM;
L-serine at a concentration between about 522 µM and 528 µM;
L-threonine at a concentration between about 1.44 mM and about 1.46 mM;
L-tryptophan at a concentration between about 162 µM and 165 µM;
L-tyrosine disodium salt at a concentration between about 692 µM and 699 µM; and
L-valine at a concentration between about 1.44 mM and about 1.46 mM.

7. The medium of claim 4 wherein the plurality of components comprises:
biotin at a concentration between about 40 nM and about 42 nM;
choline chloride at a concentration between about 36 µM and about 38 µM;
d-calcium pantothenate at a concentration between about 10 µM and about 12 µM;
folic acid at a concentration between about 11 µM and about 13 µM;
nicotinamide at a concentration between about 41 µM and about 44 µM;
pyridoxal hydrochloride at a concentration between about 24 µM and about 26 µM;
riboflavin at a concentration between about 1.38 µM and about 1.41 µM;
thiamine hydrochloride at a concentration between about 15.51 µM and about 15.67 µM;
vitamin B12 at a concentration between about 7.37 nM and about 7.38 nM;
i-inositol at a concentration between about 54.54 µM and about 55.00 µM;
calcium chloride dihydrate at a concentration between about 1.20 mM and about 1.22 mM;
magnesium sulfate heptahydrate at a concentration between about 656 µM and about 665 µM;
potassium chloride at a concentration between about 3.57 mM and about 3.63 mM;
potassium nitrate at a concentration between about 593 nM and about 594 nM;
sodium bicarbonate at a concentration between about 29.14 mM and about 29.50 mM;
sodium chloride at a concentration between about 65.24 mM and about 66.00 mM;
sodium phosphate monobasic at a concentration between about 950 µM and about 963 µM;
sodium selenite at a concentration between about 56 nM and about 58 nM; and
d-glucose at a concentration between about 20.23 mM and about 20.49 mM.

8. The medium of claim 5 wherein the plurality of components comprises:
HEPES at a concentration between about 4.8 mM and about 5 mM;
phenol red at a concentration between about 34.23 M and about 35.00 µM;
sodium pyruvate at a concentration between about 0.5 mM and about 3.5 mM;
heat inactivated AB serum at a percent volume of between about 2.60 percent and about 2.70 percent;
human serum albumin at a concentration between about 1.75 mg/mL and about 1.79 mg/mL;
insulin at a concentration between about 8.70 µg/mL and about 9.00 µg/mL;
heparin at a concentration between about 2.63 µg/mL and about 2.68 µg/mL;
erythropoietin at a concentration between about 44 ng/mL and about 45 ng/mL; and
glutamax at a concentration between about 5.26 mM and about 5.36 mM.

9. A system comprising the bioreactor with a recirculating loop and the medium of any of claims 1 to 8.

10. A medium for culturing cells optimized for use in a bioreactor with periodic partial medium exchanges or additions, the medium comprising a plurality medium components optimized for in vitro bioreactor-based manufacture of red blood cells (RBCs).

11. The medium of claim 10 wherein the plurality of components comprises:
glycine at a concentration of between about 522 µM and 528 µM;
L-alanine at a concentration of between about 425 µM and 430 µM;
L-arginine hydrochloride of between about 1.52 mM and 1.53 mM;
L-asparagine of between about 372 µM and 377 µM;
L-aspartic acid of between about 381 µM and 386 µM;
L-cystine of between about 434 µM and 440 µM;
L-glutamic acid of between about 611 µM and 618 µM;
L-histidine hydrochloride-H2O of between about 561 µM and 566 µM;
L-isoleucine at a concentration between about 1.44 mM and about 1.46 mM;
L-leucine at a concentration between about 1.44 mM and about 1.46 mM;
L-lysine at a concentration between about 1.80 mM and about 1.82 mM;
L-methionine at a concentration between about 364 µM and 368 µM;
L-phenylalanine at a concentration between about 721 µM and 728 µM;
L-proline at a concentration between about 480 µM and 485 µM;
L-serine at a concentration between about 522 µM and 528 µM;
L-threonine at a concentration between about 1.44 mM and about 1.46 mM;
L-tryptophan at a concentration between about 162 µM and 165 µM;
L-tyrosine disodium salt at a concentration between about 692 µM and 699 µM;
L-valine at a concentration between about 1.44 mM and about 1.46 mM;
biotin at a concentration between about 40 nM and about 42 nM;
choline chloride at a concentration between about 36 µM and about 38 µM;
d-calcium pantothenate at a concentration between about 10 µM and about 12 µM;
folic acid at a concentration between about 11 µM and about 13 µM;
nicotinamide at a concentration between about 41 µM and about 44 µM;
pyridoxal hydrochloride at a concentration between about 24 µM and about 26 µM;
riboflavin at a concentration between about 1.38 µM and about 1.41 µM;
thiamine hydrochloride at a concentration between about 15.51 µM and about 15.67 µM;
vitamin B12 at a concentration between about 7.37 nM and about 7.38 nM;
i-inositol at a concentration between about 54.54 µM and about 55.00 µM;
calcium chloride dihydrate at a concentration between about 1.20 mM and about 1.22 mM;
magnesium sulfate heptahydrate at a concentration between about 656 µM and about 665 µM;
potassium chloride at a concentration between about 3.57 mM and about 3.63 mM;
potassium nitrate at a concentration between about 593 nM and about 594 nM;
sodium bicarbonate at a concentration between about 29.14 mM and about 29.50 mM;
sodium chloride at a concentration between about 65.24 mM and about 66.00 mM;
sodium phosphate monobasic at a concentration between about 950 µM and about 963 µM;
sodium selenite at a concentration between about 56 nM and about 58 nM;
d-glucose at a concentration between about 20.23 mM and about 20.49 mM;
HEPES at a concentration between about 4.8 mM and about 5.0 mM;
phenol red at a concentration between about 34.23 µM and about 35.00 µM;
sodium pyruvate at a concentration between about 0.5 mM and about 3.5 mM;
heat inactivated AB serum at a percent volume of between about 2.60 percent and about 2.70 percent;
human serum albumin at a concentration between about 1.75 mg/mL and about 1.79 mg/mL;
insulin at a concentration between about 8.70 µg/mL and about 9.00 µg/mL;
heparin at a concentration between about 2.63 µg/mL and about 2.68 µg/mL;
erythropoietin at a concentration between about 44 ng/mL and about 45 ng/mL; and
glutamax at a concentration between about 5.26 mM and about 5.36 mM.

12. A medium for culturing cells, the medium comprising a plurality of components comprising:
glycine at a concentration of between about 522 µM and 528 µM;
L-alanine at a concentration of between about 425 µM and 430 µM;
L-arginine hydrochloride of between about 1.52 mM and 1.53 mM;
L-asparagine of between about 372 µM and 377 µM;
L-aspartic acid of between about 381 µM and 386 µM;
L-cystine of between about 434 µM and 440 µM;
L-glutamic acid of between about 611 µM and 618 µM;
L-histidine hydrochloride-H2O of between about 561 µM and 566 µM;
L-isoleucine at a concentration between about 1.44 mM and about 1.46 mM;
L-leucine at a concentration between about 1.44 mM and about 1.46 mM;
L-lysine at a concentration between about 1.80 mM and about 1.82 mM;
L-methionine at a concentration between about 364 µM and 368 µM;
L-phenylalanine at a concentration between about 721 µM and 728 µM;
L-proline at a concentration between about 480 µM and 485 µM;
L-serine at a concentration between about 522 µM and 528 µM;
L-threonine at a concentration between about 1.44 mM and about 1.46 mM;
L-tryptophan at a concentration between about 162 µM and 165 µM;
L-tyrosine disodium salt at a concentration between about 692 µM and 699 µM;
L-valine at a concentration between about 1.44 mM and about 1.46 mM;
biotin at a concentration between about 40 nM and about 42 nM;
choline chloride at a concentration between about 36 µM and about 38 µM;
d-calcium pantothenate at a concentration between about 10 µM and about 12 µM;
folic acid at a concentration between about 11 µM and about 13 µM;
nicotinamide at a concentration between about 41 µM and about 44 µM;
pyridoxal hydrochloride at a concentration between about 24 µM and about 26 µM;
riboflavin at a concentration between about 1.38 µM and about 1.41 µM;
thiamine hydrochloride at a concentration between about 15.51 µM and about 15.65 µM;
vitamin B12 at a concentration between about 7.37 nM and about 7.38 nM;
i-inositol at a concentration between about 54.54 µM and about 55.00 µM;
calcium chloride dihydrate at a concentration between about 1.20 mM and about 1.22 mM;
magnesium sulfate heptahydrate at a concentration between about 656 µM and about 665 µM;
potassium chloride at a concentration between about 3.57 mM and about 3.63 mM;
potassium nitrate at a concentration between about 593 nM and about 594 nM;
sodium bicarbonate at a concentration between about 29.14 mM and about 29.50 mM;
sodium chloride at a concentration between about 65.24 mM and about 66.00 mM;
sodium phosphate monobasic at a concentration between about 950 µM and about 963 µM;
sodium selenite at a concentration between about 56 nM and about 58 nM;
d-glucose at a concentration between about 20.23 mM and about 20.49 mM;
HEPES at a concentration between about 4.8 mM and about 5.0 mM;
phenol red at a concentration between about 34.23 µM and about 35 µM;
sodium pyruvate at a concentration between about 0.5 mM and about 3.5 mM;
heat inactivated AB serum at a percent volume of between about 2.60 percent and about 2.70 percent;
human serum albumin at a concentration between about 1.75 mg/mL and about 1.79 mg/mL;
insulin at a concentration between about 8.70 µg/mL and about 9.00 µg/mL;
heparin at a concentration between about 2.63 µg/mL and about 2.68 µg/mL;
erythropoietin at a concentration between about 44 ng/mL and about 45 ng/mL;
glutamax at a concentration between about 5.26 mM and about 5.36 mM; and
an iron source.

13. The medium of claim 12 wherein the iron source comprises holo-transferrin at a concentration between about 0.68 mg/mL and 0.95 mg/mL.

14. The medium of claim 12 or claim 13 wherein the iron source comprises iron (III) citrate at a concentration of between 1 µg/mL and 500 µg/mL.

15. A method of formulating a medium for use in a bioreactor with periodic partial medium exchanges, the method comprising:
determining average depletion rates for a plurality of factors in the medium when used in the bioreactor with partial medium exchanges;
formulating the medium based on the average depletion rates to supply the plurality of factors to allow for cell growth and proliferation while maintaining a homeostatic culture environment;
wherein the medium comprises iron (III) citrate at a concentration of between 1 µg/mL and 500 µg/mL.
